# EUROPEAN PATENT APPLICATION

(11) **EP 4 606 399 A1**
(43) Date of publication of application: **27.08.2025**
(21) Application number: 24165535.6
(22) Date of filing: 22.03.2024
(51) Int. Cl.: A61M 1/02

(54) **BLOOD FILTRATION SYSTEM**

(30) Priority: 20.02.2024 TW 113105877
(71) Applicant: Sangtech Lab Inc., Taipei (TW)
(72) Inventor: JUAN, Tien-Chu, Taipei (TW); LIANG, Cheng-Sheng, Taipei (TW); CHUANG, Pei-Chieh, Taipei (TW); CHEN, Yong-Guei, Taipei (TW)
(74) Representative: Becker, Eberhard

(57) **Abstract**

Disclosed is a blood filtration system (100) including a first blood storage bag (110), a second blood storage bag (120), a first pipeline (130), a filter (140) and a second pipeline (150). The first blood storage bag (110) is used to store blood (B). The first pipeline (130) is connected between the first blood storage bag (110) and the second blood storage bag (120). The filter (140) is configured on the first pipeline (130). The filter (140) is suitable for blood (B) to pass through via the first pipeline (130) from the first blood storage bag (110) to the second blood storage bag (120). The second pipeline (150) is connected between the first blood storage bag (110) and the second blood storage bag (120). The second pipeline (150) is suitable for air in the second blood storage bag (120) to pass through to reach the first blood storage bag (110).

## Description

### BACKGROUND

### Technical Field

The disclosure relates to a filtration system, and in particular to a blood filtration system.

### Description of Related Art

Blood donation usually requires transfusing a donor's blood into a whole blood bag first, and then allowing the blood to pass through a pipeline disposed with a filter to filter the white blood cells and other substances in the blood, and subsequently transfusing the filtered blood into a collection bag through the pipeline. In the process of transfusing the filtered blood into the collection bag, it is required that the air in the collection bag be discharged through the pipeline. However, since the pipeline contains the blood being transfused into the collection bag, the air in the collection bag cannot be easily discharged through the pipeline automatically.

Thus, it usually requires adding an air discharge bypass to the pipeline in parallel with the filter. When the air in the collection bag is to be discharged, the flow path passing through the filter is first blocked by a clamping device, allowing the air in the collection bag to enter the pipeline and be discharged into the blood bag at upstream through the air discharge bypass by manual squeezing of the collection bag under the condition of stopping blood transfusion into the collection bag. This design results in a complicated pipeline structure and the requirement for manual squeezing for air discharge, which is very time-consuming and cumbersome.

### SUMMARY

The disclosure provides a blood filtration system, which simplifies a pipeline structure and facilitate an automatic air discharge.

The blood filtration system of the disclosure includes a first blood storage bag, a second blood storage bag, a first pipeline, a filter and a second pipeline. The first blood storage bag is used to store blood. The first pipeline is connected between the first blood storage bag and the second blood storage bag. The filter is configured on the first pipeline. The filter is suitable for blood to pass through via the first pipeline from the first blood storage bag to the second blood storage bag. The second pipeline is connected between the first blood storage bag and the second blood storage bag. The second pipeline is suitable for the air in the second blood storage bag to pass through to reach the first blood storage bag.

In an embodiment of the disclosure, the aforementioned first pipeline and the second pipeline are independent of each other.

In an embodiment of the disclosure, the aforementioned first pipeline has a first inlet end and a first outlet end opposite to each other. The first inlet end is directly connected to the first blood storage bag, and the first outlet end is directly connected to the second blood storage bag. The second pipeline has a second inlet end and a second outlet end opposite to each other. The second inlet end is directly connected to the second blood storage bag, and the second outlet end is directly connected to the first blood storage bag.

In an embodiment of the disclosure, each of the aforementioned first inlet end and the second outlet end is connected to a different position of the first blood storage bag, and each of the first outlet end and the second inlet end is connected to a different position of the second blood storage bag.

In an embodiment of the disclosure, the aforementioned blood filtration system further includes a one-way valve connected to the second pipeline. The one-way valve is suitable for the air in the second blood storage bag to pass through via the second pipeline to reach the first blood storage bag. The one-way valve is used to prevent the blood in the first blood storage bag from flowing into the second blood storage bag via the second pipeline.

In an embodiment of the disclosure, the aforementioned one-way valve is located in the first blood storage bag.

In an embodiment of the disclosure, the aforementioned one-way valve is integrally connected to the first blood storage bag.

In an embodiment of the disclosure, a material of the aforementioned one-way valve is a same material as the first blood storage bag.

In an embodiment of the disclosure, the aforementioned one-way valve is connected to the first blood storage bag by high-frequency welding.

In an embodiment of the disclosure, the aforementioned one-way valve includes a valve body and a blocking structure. The blocking structure is used to block a flow path of the valve body and is suitable to be changed to an open state to open the flow path of the valve body.

Based on the above, between the first blood storage bag and the second blood storage bag in the blood filtration system of the disclosure, not only the first pipeline is disposed to transfuse blood from the first blood storage bag into the second blood storage bag, the second pipeline is also disposed to discharge air from the second blood storage bag to the first blood storage bag. In other words, a blood transfusion and an air discharge between the first blood storage bag and the second blood storage bag are carried out separately and via different pipelines. A flow path for the blood transfusion and a flow path for the air discharge in the blood filtration system do not share a same pipeline at all. Instead, two independent pipelines are disposed. Accordingly, during a process of the blood transfusion from the first blood storage bag into the second blood storage bag through the first pipeline, a pressure in the second blood storage bag rises as the blood in the second blood storage bag increases, allowing the air in the second blood storage bag to flow to the first blood storage bag through the second pipeline automatically. As a consequence, the blood filtration system of the disclosure simplifies a pipeline structure and facilitates an automatic air discharge.

### BRIEF DESCRIPTION OF THE DRAWINGS

FIG. 1 is a schematic diagram of a blood filtration system in an embodiment of the disclosure.
FIGs. 2A to 2C illustrate how the blood filtration system in FIG. 1 functions.
FIG. 3A is a cross-sectional schematic diagram of a one-way valve in FIG. 1.
FIG. 3B illustrates a damage of a blocking structure in FIG. 3A.

### DESCRIPTION OF THE EMBODIMENTS

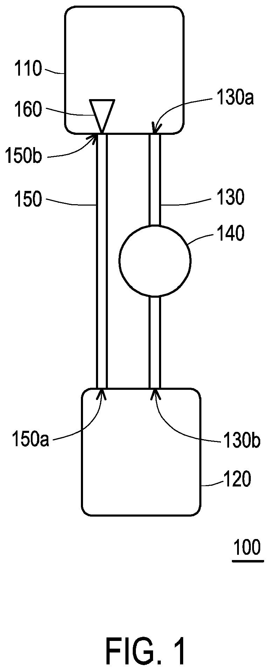

FIG. 1 is a schematic diagram of a blood filtration system in an embodiment of the disclosure. Please refer to FIG. 1. A blood filtration system 100 in this embodiment includes a first blood storage bag 110, a second blood storage bag 120, a first pipeline 130, a filter 140 and a second pipeline 150. The first pipeline 130 is connected between the first blood storage bag 110 and the second blood storage bag 120. The filter 140 is configured on the first pipeline 130. The second pipeline 150 is connected between the first blood storage bag 110 and the second blood storage bag 120. The first pipeline 130 and the second pipeline 150 are independent of each other. The blood filtration system 100 may be used to filter blood from a blood donor or blood for other purposes. This is not limited by the disclosure.

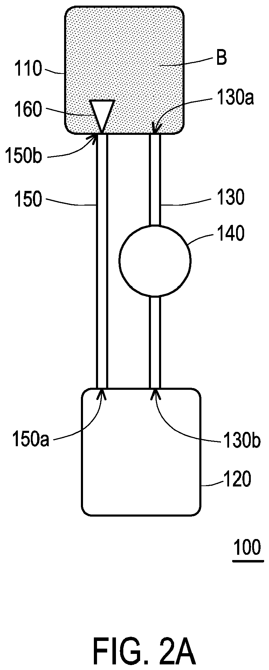

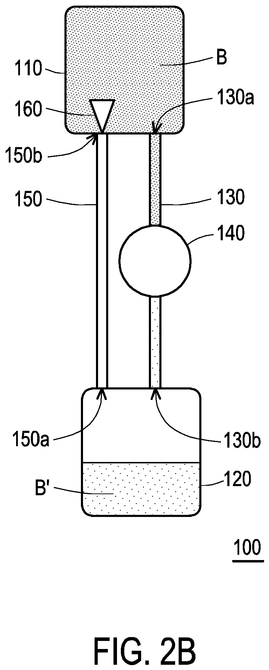

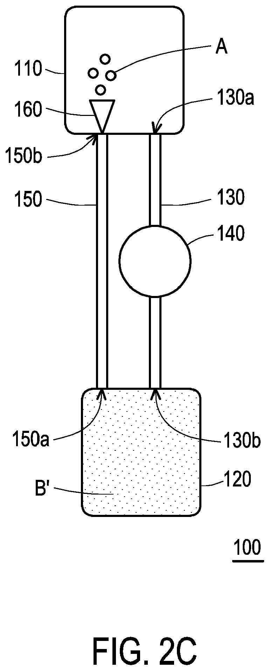

FIGs. 2A to 2C illustrate a mode of operation of the blood filtration system in FIG. 1. In this embodiment, the first blood storage bag 110 is, for example, a whole blood bag for storing blood B from a blood donor as shown in FIG. 2A. The blood B may pass from the first blood storage bag 110 to the second blood storage bag 120, which serves as a collection bag, through the filter 140 via the first pipeline 130 as shown in FIG. 2B by manual squeezing of the first blood storage bag 110 or other proper methods. The filtered blood is marked as B' in FIG. 2B. The filter 140 is used, for example, to filter white blood cells and/or other substances in the blood. A detailed filtering structure and a detailed filtering method of the filter 140 are known in the art and will not be described herein. The second pipeline 150 is suitable for the air in the second blood storage bag 120 to pass through to reach the first blood storage bag 110 as shown in FIG. 2C, and the air reaching the first blood storage bag 110 is marked as A in FIG. 2C.

As described above, between the first blood storage bag 110 and the second blood storage bag 120 in the blood filtration system 100 in this embodiment, not only the first pipeline 130 is disposed to transfuse blood from the first blood storage bag 110 into the second blood storage bag 120, the second pipeline 150 is also disposed to discharge air from the second blood storage bag 120 to the first blood storage bag 110. In other words, a blood transfusion and an air discharge between the first blood storage bag 110 and the second blood storage bag 120 are carried out separately and via different pipelines. A flow path for the blood transfusion and a flow path for the air discharge in the blood filtration system 100 do not share a same pipeline at all. Instead, two independent pipelines are disposed.

Accordingly, during a process of the blood transfusion from the first blood storage bag 110 into the second blood storage bag 120 through the first pipeline 130, a pressure in the second blood storage bag 120 rises as the blood in the second blood storage bag 120 increases, allowing the air in the second blood storage bag 120 to flow to the first blood storage bag 110 through the second pipeline 150 automatically due to Pascal's principle. As a consequence, the blood filtration system 100 in this embodiment simplifies a pipeline structure and facilitates an automatic air discharge.

Specifically, the first pipeline 130 in this embodiment has a first inlet end 130a and a first outlet end 130b opposite to each other. The first inlet end 130a is directly connected to the first blood storage bag 110, and the first outlet end 130b is directly connected to the second blood storage bag 120. The second pipeline 150 has a second inlet end 150a and a second outlet end 150b opposite to each other. The second inlet end 150a is directly connected to the second blood storage bag 120, and the second outlet end 150b is directly connected to the first blood storage bag 110.

Furthermore, each of the first inlet end 130a and the second outlet end 150b is connected to a different position of the first blood storage bag 110. Each of the first outlet end 130b and the second inlet end 150a is also connected to a different position of the second blood storage bag 120. As a consequence, between the first blood storage bag 110 and the second blood storage bag 120, the first pipeline 130 and the second pipeline 150 are independent of each other as described above and do not share the pipeline at all.

In detail, in this embodiment, the blood filtration system 100 further includes a one-way valve 160, which is connected to the second pipeline 150. The one-way valve 160 is suitable for the air in the second blood storage bag 120 to pass through via the second pipeline 150 to reach the first blood storage bag 110. The one-way valve 160 is used to prevent the blood in the first blood storage bag 110 from flowing into the second blood storage bag 120 via the second pipeline 150 in order to avoid the blood obstructing the air discharge to the first blood storage bag 110 via the second pipeline 150. A structural design and a mode of operation of the one-way valve 160 for restricting a one-way flow are known in the art and will not be described herein.

Furthermore, a material of the one-way valve 160 in this embodiment is, for example, a same material as the first blood storage bag 110. The one-way valve 160 is located in the first blood storage bag 110 and integrally connected to the first blood storage bag 110 by a high-frequency welding process or other suitable processes. As a result, the one-way valve 160 may be manufactured and configured through a simple manufacturing process, and the one-way valve 160 is accommodated in the first blood storage bag 110 without the need of disposing an additional cavity body to accommodate the one-way valve 160. The materials of the first blood storage bag 110 and the one-way valve 160 may be PVCs or other suitable types of plastic materials. This is not limited by the disclosure. In addition, materials of the second blood storage bag 120, the first pipeline 130, and the second pipeline 150 may be PVCs or other suitable types of plastic materials as well. In other embodiments, the one-way valve 160 may be configured outside the first blood storage bag 110 and at a suitable position on the second pipeline 150. This is not limited by the disclosure.

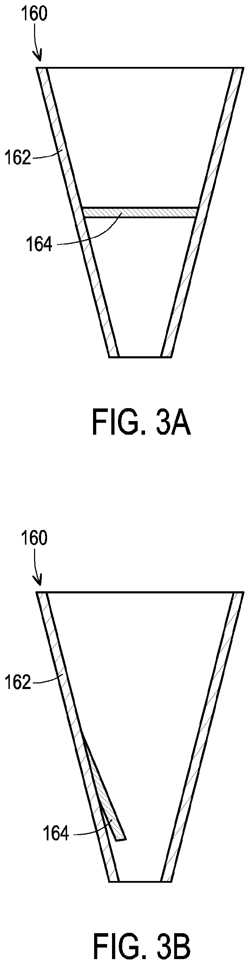

FIG. 3A is a cross-sectional schematic diagram of a one-way valve in FIG. 1. FIG. 3B illustrates a damage of a blocking structure in FIG. 3A. Please refer to FIG. 3A. In this embodiment, the one-way valve 160 includes a valve body 162 and a blocking structure 164. The blocking structure 164 is used to block a flow path of the valve body 162, and the blocking structure 164 is suitable to be changed to an open state to open the flow path of the valve body 162. For example, the blocking structure 164 is a flow path blocking material configured in the valve body 162 as shown in FIG. 3A. The valve body 162 may be damaged as the valve body 162 undertakes a force applied by a user, leaving the flow path of the valve body 162 open as shown in FIG. 3B. In other embodiments, the blocking structure 164 may be a clamping element (e.g., a plastic clamp), which is configured at an upstream or a downstream of the valve body 162 to clamp a pipeline at the upstream or the downstream of the valve body 162 to achieve an effect of blocking the flow path. The blocking structure 164 may be loosened as the user applies a force to the clamping element, thereby opening the flow path of the valve body 162. As a result, it is ensured that the one-way valve 160 is activated only when an air discharging operation is required, and an unintended activation of the one-way valve 160 when an air discharging operation is not performed is further avoided. FIGs. 3A and 3B simply illustrate schematic structural shapes of the valve body 162 and the blocking structure 164, which may actually be of other suitable appearances. This is not limited by the disclosure.

In summary, between the first blood storage bag and the second blood storage bag in the blood filtration system of the disclosure, not only the first pipeline is disposed to transfuse blood from the first blood storage bag into the second blood storage bag, the second pipeline is also disposed to discharge air from the second blood storage bag to the first blood storage bag. In other words, a blood transfusion and an air discharge between the first blood storage bag and the second blood storage bag are carried out separately and via different pipelines. A flow path for the blood transfusion and a flow path for the air discharge in the blood filtration system do not share a same pipeline at all. Instead, two independent pipelines are disposed. Accordingly, during a process of the blood transfusion from the first blood storage bag into the second blood storage bag through the first pipeline, a pressure in the second blood storage bag rises as the blood in the second blood storage bag increases, allowing the air in the second blood storage bag to flow to the first blood storage bag through the second pipeline automatically. As a consequence, the blood filtration system of the disclosure simplifies a pipeline structure and facilitates an automatic air discharge.

## Claims

1. A blood filtration system (100), comprising:
a first blood storage bag (110) configured to store blood (B);
a second blood storage bag (120);
a first pipeline (130) connected between the first blood storage bag (110) and the second blood storage bag (120);
a filter (140) configured on the first pipeline (130), the filter (140) being adaptable for the blood (B) to pass through via the first pipeline (130) from the first blood storage bag (110) to the second blood storage bag (120); and
a second pipeline (150) connected between the first blood storage bag (110) and the second blood storage bag (120), the second pipeline (150) being adaptable for air in the second blood storage bag (120) to pass through to reach the first blood storage bag (110).

2. The blood filtration system (100) of claim 1, wherein the first pipeline (130) and the second pipeline (150) are independent of each other.

3. The blood filtration system (100) of claim 1, wherein the first pipeline (130) has a first inlet end (130a) and a first outlet end (130b) opposite to each other, the first inlet end (130a) being directly connected to the first blood storage bag (110), and the first outlet end (130b) being directly connected to the second blood storage bag (120), wherein the second pipeline (150) has a second inlet end (150a) and a second outlet end (150b) opposite to each other, the second inlet end (150a) being directly connected to the second blood storage bag (120), and the second outlet end (150b) being directly connected to the first blood storage bag (110).

4. The blood filtration system (100) of claim 3, wherein each of the first inlet end (130a) and the second outlet end (150b) is connected to a different position of the first blood storage bag (110), and each of the first outlet end (130b) and the second inlet end (150a) is connected to a different position of the second blood storage bag (120).

5. The blood filtration system (100) of claim 1, further comprising a one-way valve (160) connected to the second pipeline (150), wherein the one-way valve (160) is adaptable for the air in the second blood storage bag (120) to pass through via the second pipeline (150) to reach the first blood storage bag (110), the one-way valve (160) being configured to prevent the blood (B) in the first blood storage bag (110) from flowing into the second blood storage bag (120) via the second pipeline (150).

6. The blood filtration system (100) of claim 5, wherein the one-way valve (160) is located in the first blood storage bag (110).

7. The blood filtration system (100) of claim 5, wherein the one-way valve (160) is integrally connected to the first blood storage bag (110).

8. The blood filtration system (100) of claim 5, wherein a material of the one-way valve (160) is a same material as the first blood storage bag (110).

9. The blood filtration system (100) of claim 5, wherein the one-way valve (160) is connected to the first blood storage bag (110) by high-frequency welding.

10. The blood filtration system (100) of claim 5, wherein the one-way valve (160) comprises a valve body (162) and a blocking structure (164), wherein the blocking structure (164) is configured to block a flow path of the valve body (162), and the blocking structure (164) is adaptable to be changed to an open state to open the flow path of the valve body (162).
